# EUROPEAN PATENT APPLICATION

(11) **EP 4 431 041 A1**
(43) Date of publication of application: **18.09.2024**
(21) Application number: 23161365.4
(22) Date of filing: 13.03.2023
(51) Int. Cl.: A61B 34/20

(54) **MARKER SYSTEM**

(71) Applicant: Incremed AG, 8008 Zurich (CH)
(72) Inventor: WITTMANN, Frieder, 8057 Zürich (CH); PETER, Ueli, 8057 Zürich (CH); BEHM, Pascal, 8057 Zürich (CH); KRAUTZ, Christoph, 8057 Zürich (CH); KÖPFLI, Fabia, 8604 Volketswil (CH); BAY, Till, 3400 Burgdorf (CH)
(74) Representative: Liebetanz, Michael

(57) **Abstract**

A marker system comprises a marker device (100) having a marker body (110) with two to five marker surfaces (111, 112, 113) wherein each two of these marker surfaces have a common edge (114) wherein the planes of these marker surfaces meet at said common edge (114) at a blunt angle, wherein each marker surface comprises a marker symbol (121, 122, 123), an augmented reality head-mounted display (200) for a person (50) incorporating a camera (205), and a control unit (600) connected to the augmented reality head-mounted display (200) and the camera (205), wherein the camera (205) is mainly aligned in the user's line of sight (290) and wherein the control unit is configured to calculate the position and orientation of the marker device based on receiving images from the camera (205) including the marker symbols (121, 122, 123).

## Description

### TECHNICAL FIELD

The present invention relates to a marker system comprising: a marker device having a marker body with at least two marker surfaces wherein each two of these marker surfaces have a common edge wherein the planes of these marker surfaces meet at said common edge at a blunt angle, wherein each marker surface comprises a marker symbol, a camera oriented to take pictures of the marker surfaces, and a control unit connected to the camera, wherein the control unit is configured to calculate the position of the tip of the marker device based on receiving images from the camera including the marker symbols.

### PRIOR ART

Such a marker system is disclosed in an article by Wu, P-C; Wang, R; Kin, K; Twigg, C; Han, S; Yang, M-H and Chien, S-Y, which was published with the title "DodecaPen: Accurate 6DoF Tracking of a Passive Stylus" in UIST 2017, October 22-25, 2017, Pages 365-374, Quebec City, QC, Canada (DOI: https://doi.org/10.1145/3126594.3126664). It discloses a system for real-time six degrees of freedom (6DoF) tracking of a passive stylus that achieves submillimeter accuracy, which is suitable for writing or drawing in mixed reality applications. It requires a monocular camera, a dodecahedron and binary square markers on the dodecahedron surfaces. The camera is mounted on the user's desk. The article acknowledges that tracking from sparse constraints from a binary square marker alone does not deliver sufficient accuracy or robustness for tracking a pen for writing and drawing. By switching from cubes to a dodecahedron as the tracked object, at least two planes are visible in most cases, eliminating the ambiguity of the Perspective-n-Point approach.

Such a system is cumbersome and needs a dodecahedron to have always at least two visible planes of the dodecahedron to evaluate the binary symbols on these planes.

In order to improve results in surgery, it is a felt need to accelerate the procedures and as such support the surgeon with images of the patient organs or bones prepared beforehand and updated during the surgical intervention as well as providing navigational information during surgery steps such as drilling or cutting.

US 10,825,563 discloses an augmented reality system aligning image data of a patient with actual views of the patient using an optical code affixed to the patient, where a pattern of further markers are affixed to the patient relative to a position of said optical code and after having calculated the position of the pattern of said markers in the 3D space, registering the position of the inner layer of the patient in the 3D space and displaying said inner layer onto actual views of the patient. The system includes an augmented reality head display for the person using the system.

US 9,892,564 discloses a control method for an augmented reality system, wherein the augmented reality headset hides a virtual cursor and/or a virtual user interface, when it is determined that the focal orientation of the augmented reality headset is focused on the patient.

### SUMMARY OF THE INVENTION

Based on the prior art it is an object of the present invention to improve gathering of physical data as e.g. bone surface of the patient. Another object of the present invention is to simplify the use of surgical tools such as drill guides.

The invention is based on the insight that in surgical applications, the surgeon and its team often blocks the line of sight of a camera fixed somewhere in the room, even if the camera is aligned with a light source illuminating the surgical field.

A marker system according to the invention is realized with the features of claim 1.

Such a marker system can be used in connection with a tip at the end of a rod, i.e. like a stylus, defining the position of the tip vis-à-vis the marker surfaces to register a surface of the patient, especially a bone surface as e.g. a vertebral surface. Such data gathering and registration vis-à-vis the data gathering stylus allows a visualization of the bone structure in the following surgical intervention in the HMD.

The marker system can preferably also comprise in an exchangeable alternative a holder defining a direction vis-à-vis the marker surfaces. Then the holder can comprise or be attached to a drill guide or screwdriver sleeve to allow the user to visualize in the augmented reality setup of the HMD the current direction of the drill guide or screwdriver sleeve. In both cases the knowledge of the position of the holder also enables the marker system to visualize the advance of a drill or a screw in the bone material of a patient.

Compared to the prior art, the marker system according to the invention has several advantages as:
- Optimised volume/size for two to five markers with fairly larger size of the detected surface
- Design optimises visibility of at least two of the markers when the tool is used.

Such a use comprises movements with tilting and rotations of the marker system held and used by a surgeon and according to the specific choice of the marker surfaces, he is not obliged to pay specifically attention to the orientation of the marker system he uses when he has internalised holding the marker system in a general orientation of the marker surfaces towards his face. This is true for a monocular camera at the HMD, but can be improved with a binocular camera with the left and right cameras attached outside the center line of the HMD, especially in a distance equivalent to the IPD of the user (IPD = inter-pupillary distance).

When the marker device has a tip at the end of a rod, it can be used to register the position of the surface touched by the tip. Any body surface can be acquired with sweeping the tip pver the surface, while the control unit is configured to interpolate any regions between the line movements of the tip and e.g. indicating where additional registering sweeps are necessary.

The preferred marker device has one single umbrella shaped surface with two to five marker surfaces. It is also possible to provide three marker surfaces positioned one above the other in longitudinal direction on the above mentioned rod.

The marker device can comprise a holder defining a predetermined direction vis-à-vis the marker surfaces. Such a holder can have one or more positioning sockets at the underside of the marker body, wherein the positioning socket(s) is/are configured to accommodate a positioning stick defining a predetermined positioning direction vis-à-vis the three marker surfaces. Thus, the marker device can be attached to a positioning stick attached at a body or positioned in a hole in the body.

The blunt angle at which the planes of the marker surfaces meet at a common edge is greater than 180 degrees and the planes create an umbrella like shape, or is smaller than 180 degrees and the planes create an upturned umbrella like shape.

The dihedral angle between the top surface and the respective side surface of the polyhedron can be between 205 degrees and 235 degrees, preferably 225 degrees for the umbrella like shape or between 125 degrees and 155 degrees, preferably 135 degrees for the upturned umbrella like shape, wherein the polygonal angle at the vertex is between 110 degrees and 125 degrees, preferably 120 degrees.

The marker surfaces can have specific orientations as mentioned above, i.e. being a trihedron or quadhedron. They can also be in the shape of a trigonal or quadronal hosohedron, i.e. a tessellation of lunes on a spherical surface.

The control unit can be configured to access a previously acquired 3D image of a body and to calculate and to display said 3D image in at least one of an axial and a sagittal view in a side area of the field of view of the augmented reality head-mounted display, and to calculate and display a virtual representation of the position and orientation of the marker device. The body can be any item for which the surface is to be registered and where e.g. a hole has to be drilled at a predetermined place and orientation. Such a body can be a bone, especially a vertebra. Each AR HMD has a FOV (field of view). A side area of such a FOV is usually considered to be at about 50% off the center from the field of view. In view of the bifocal view of the user, this can be preferably be to the left and/or to the right of the center of the FOV.

The control unit can be configured to display a previously determined shape of intended intervention in the said view(s) of the side area together with the virtual representation of the position and orientation of the marker device. This especially enables to use as the previously determined shape of intended intervention a double conical frustum displayed on the AR HMD.

The control unit can be configured to calculate and to display the virtual representation of the marker device in a view from above on the conical frustum, providing navigational information or rotational / directional and positional error in a further side area, wherein preferably the view(s) from above comprise(s) a center indication representating the frustum axis and/or the virtual planned entry point and the virtual representation of the marker device reflecting the angle or distance deviation from the frustum axis and the virtual planned entry point, respectively.

The marker symbols can especially be chosen from the group comprising 2D barcodes, filled or line polygons, identical or different geometrical shapes.

A method of providing visual indication for a planned drill entry point into a body and planned drill direction comprises:
- having a marker system according to any one of the marker system claims,
- having a 3D image modality of the body,
- determining the planned drill direction and planned drill entry point in the body as virtual data,
- registering real world position of the body by collecting surface points with a marker device of the marker system,
- displaying axial and sagittal cuts of the 3D image modality of the body for validating the registered position with the marker device of the marker system on a augmented reality head-mounted display of the marker system, and
- displaying virtual navigation elements reflecting rotation and distance error from the predetermined planned drill direction and planned drill entry point on the augmented reality head-mounted display of the marker system.

Within such a method, the determination of the planned drill direction and planned drill entry point in the body as virtual data can comprise providing a virtual double conical frustum to be displayed in connection with the axial and sagittal cuts of the 3D image modality.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an example augmented reality (AR) environment in which a user with an augmented reality head-mounted display holds a marker according to an embodiment of the invention in his left hand;
- Fig. 2: shows the back of a marker according to an embodiment of the invention with dashed lines;
- Fig. 3: shows a bone with a positioning stick embedded in an appropriate drilled channel and schematically the front side of the marker of Fig. 2;
- Fig. 4: shows the bone of Fig. 3 with the schematically shown marker of Fig. 2 positioned on the positioning stick with marking surfaces as the marker of Fig. 1;
- Fig. 5: shows a schematic front side view of the marker as illustrated in Fig. 1;
- Fig. 6: shows a schematic front side view of a further marker with three identical markings;
- Fig. 7: shows a schematic front side view of a marker with an in-line distribution of the marking surfaces;
- Fig. 8A: shows a schematic front side view of an umbrella shaped marker with an attachment portion;
- Fig. 8B: shows the marker of Fig. 8A rotated by an angle in a forward rotation;
- Fig. 9: shows the marker of Fig. 8A rotated by an angle in a counterclockwise rotation;
- Fig. 10: shows the marker of Fig. 8A rotated by an angle in a clockwise rotation;
- Fig. 11A: shows a schematic front side view of a further upturned umbrella shaped marker with an attachment portion;
- Fig. 11B: shows the marker of Fig. 11A rotated by an angle in a backward rotation;
- Fig. 12: shows the marker of Fig. 11A rotated by an angle in a counterclockwise rotation;
- Fig. 13: shows the marker of Fig. 11A rotated by an angle in a clockwise rotation;
- Fig. 14: shows a schematic view from above on a further marker having two marking surfaces;
- Fig. 15: shows a schematic view from above on a further marker having four marking surfaces;
- Fig. 16: shows a schematic view from above on a further marker having five marking surfaces;
- Fig. 17: shows the user of Fig. 1 in an operating room with a patient lying on his stomach with his dorsal vertebrae visible;
- Fig. 18: shows the view of the user through the augmented reality head-mounted display (AR-HMD) holding a drill guide with an attached marker in his right hand near a dorsal vertebrae and two virtual images of said dorsal vertebrae with the actual axis as determined by the current position of the drill guide;
- Fig. 19: shows a flowchart of a method for using the system according to the invention; and
- Fig. 20: shows an overview of a computer system used in connection with such a system.

### DESCRIPTION OF PREFERRED EMBODIMENTS

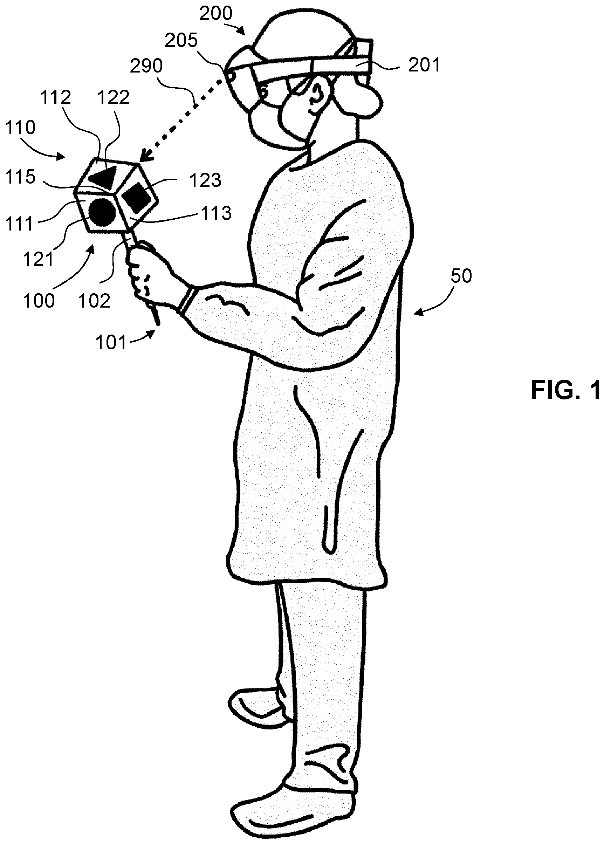

Fig. 1 shows an example augmented reality (AR) environment in which a user 50 with an augmented reality head-mounted display 200, in the following named AR HMD, holds a marker device 100 according to an embodiment of the invention in his left hand. User 50 is usually a surgeon and he uses the marker device 100 in order to scan the bone surface of a patient. He uses therefore the tip 101 of the marker device 100 and sweeps it over the bone surface in all relevant details. The marker device 100 has a trihedron as marker body 110. A trihedron is a geometric figure composed of three planes 111, 112 and 113 meeting at a single vertex 115. The three planes are the first marker surface 111, the second marker surface 112 and the third marker surface 113. Each marker surface 111, 112 and 113 have a first, second and third marker symbol 121, 122 and 123, respectively. In the embodiment of the marker device 100 of Fig. 1 these symbols are a circle, a square or a rhombus and a triangle, wherein the shapes are filled with a different colour than the remaining surface of the marker surface 111, 112 and 113, respectively. These first, second and third marker symbols 121, 122 and 123 can also be 2D barcodes or any shape which can be recognized by an evaluation of an image taken by a camera 205.

The trihedron can be replaced in other embodiments by a polyhedron with two to five marker surfaces.

There is no need for a direct line of sight (LOS) between the tip 101 and the camera. It is sufficient that the line of sight 290 between the camera 205 and the marker body 110 is free and that the marker body 110 is sufficiently illuminated.

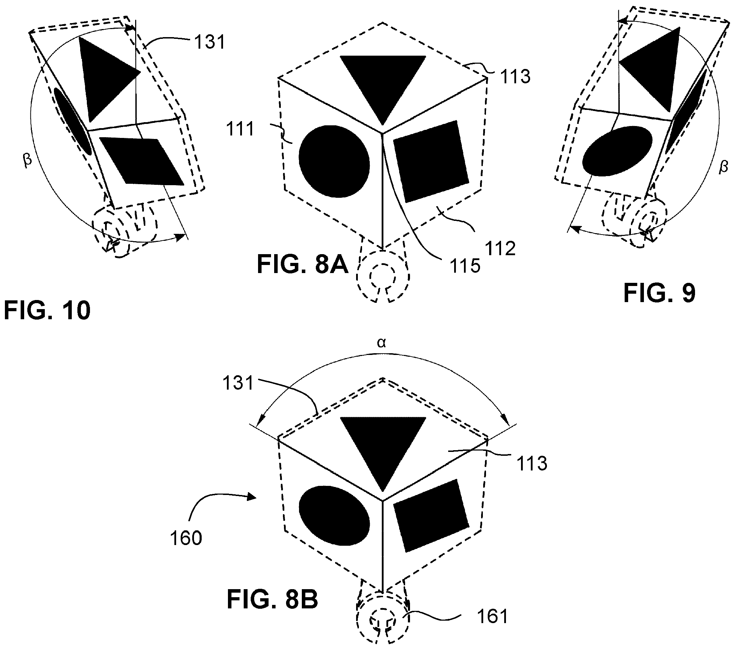

The camera 205 is a monocular camera embedded in the front portion AR HMD 200, e.g. above and between the eyes of the user 50, when he has affixed the AR HMD 200 with a strap 201 around his head. The user 50 is holding the marker device with the trihedron in his viewing direction, ensuring that when he sees two or three of the three marker surfaces 111, 112 and 113, respectively, then also the camera 205 has a direct line of sight 290 towards these surfaces and can take images. As will be explained later on, a control unit 600 connected with the camera 205, preferably integrated or over wireless transceivers, is receiving the data of the images by the camera 205 and calculates the position of the tip 101 of the marker device 100 based on the position and orientation of the trihedron, wherein the geometric relationship between different points of the symbols 121, 122 and 123 and their relationship to the tip 101 are used. This scanning of e.g. the bone surface allows the construction of a virtual image of the underlying bone in its outer physical boundaries. In other embodiments the camera can also be a binocular camera, e.g. a left camera positioned on the left half of the AR HMD and a right camera positioned on the right half of the AR HMD. Then as will be seen in connection with Fig. 8A to 13 the positioning of the marker surfaces of the handheld marker by the surgeon is even less important since the two cameras will have a LOS (line of sight) since the surgeon has this line of sight with his own eyes.

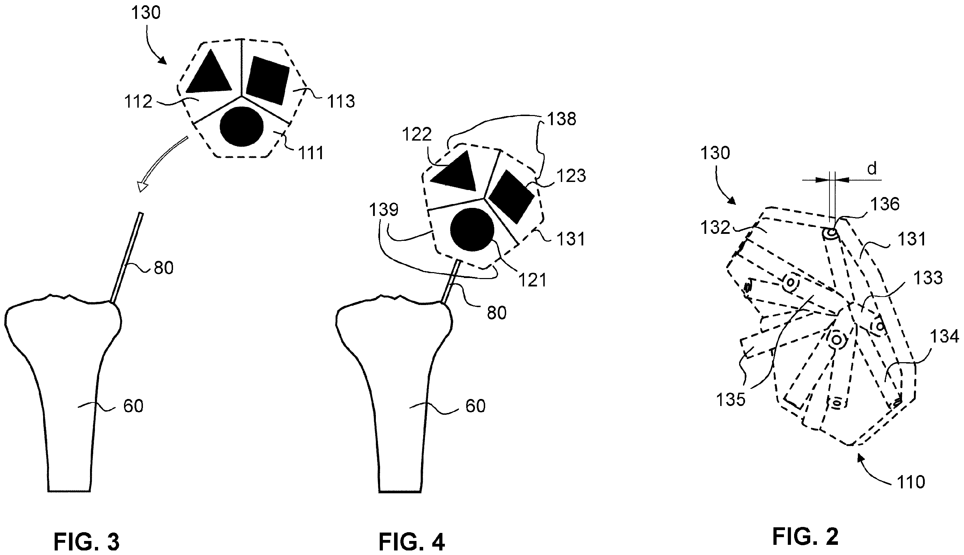

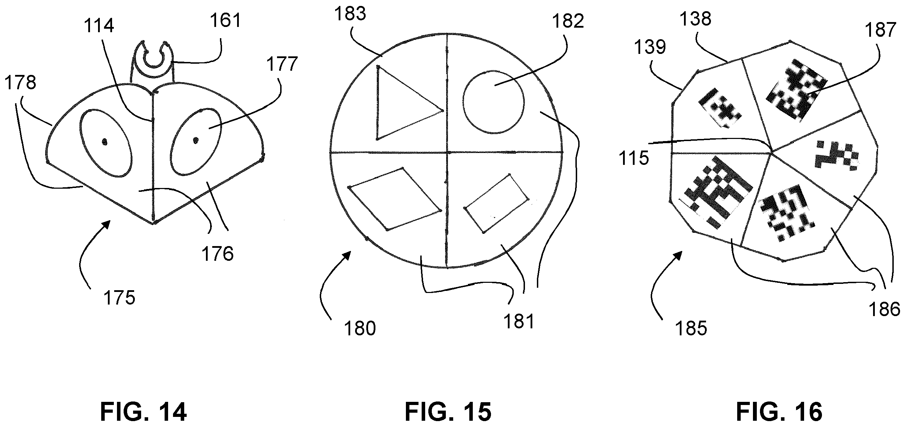

Fig. 2 shows the back of a marker device, here a marker symbol carrier 130 according to an embodiment of the invention with dashed lines. Several embodiments are shown in dashed lines to demonstrate that the borders and edges of these parts can be provided with a different shape without leaving the scope of protection. The disclosure also comprises the shape as disclosed when the dashed lines are continued as fully drawn lines. The marker device according to Fig. 2 only comprises the marker body 110 with its polygonal shaped surrounding edges 131. At the underside 132 of the marker body 110 several socket contacts 133 and 134 are attached. There are three corner socket contacts 134 under the delimiting edge 114 and three middle socket contacts 133 under the middle of the underside 132 of the trihedron. Finally, there are three outside socket contacts 135 which are oriented in a 45-degree angle under the respective middle socket contacts 133. Each of these socket contacts 133, 134, 135 are cylindrical sleeves with an internal cavity of predetermined length, allowing to accommodate a positioning stick 80. In other embodiments a different number of sockets can be chosen, e.g. one in every corner; and the marker symbol carrier can also be a carrier with a different number of marker surfaces as shown in Fig. 14, 15 and 16.

Fig. 3 shows a bone 60 with a positioning stick 80 embedded in an appropriate drilled channel in the bone 60 and schematically the front side of the marker of Fig. 2 in the distance from said stick. Fig. 4 shows the bone 60 of Fig. 3 with the schematically shown marker of Fig. 2 positioned on said positioning stick 80 with marking surfaces as are shown on the marker of Fig. 1. It is also possible to have such a positioning stick 80 glued or clipped to a bone portion. The main point of use is to determine position and orientation of this said bone portion, indicated by the predetermined length and orientation of the positioning stick 80 in relationship to the marker.

When a positioning stick 80 of predetermined length is pushed into a borehole in bone 60, then positioning of a marker symbol carrier 130 on this positioning stick 80 allows to reference the position and orientation of positioning stick 80 in the bone 60. In the embodiment shown the marker symbols 121, 122 and 123 are identical to the marker symbols of embodiment Fig. 1 and can therefore clearly be exchanged with other marker symbols.

The marker symbol carrier 130 has a polygonal outer edge surface 131 which is characterized in this embodiment by having a baseline 138, being in a view from above oriented vertical to the corresponding opposite delimiting edge 114. This baseline has then two side edges 139 leading to the joining delimiting edge lines 114 with the other two surfaces of the trihedron. These side edge surfaces 139 meeting at the delimiting edge 114 can be in one plane, being vertical to this delimiting edge 114 or form an angle in the view from above of less than 180°.

In other embodiments it is possible to replace the three marker surfaces of the trihedron by a trigonal hosohedron, i.e. a tessellation of lunes on a spherical surface, i.e. in this case each surface 111, 112 and 113 is a portion of a spherical lune, while the delimiting edge 114 on both sides are part of great circles. Then the surfaces 111, 112 and 113 are not flat but curved. It is then possible to provide the symbols 121, 122 and 123 still on a flat surface affixed to the lune surfaces or integrate the symbols into the lune surfaces.

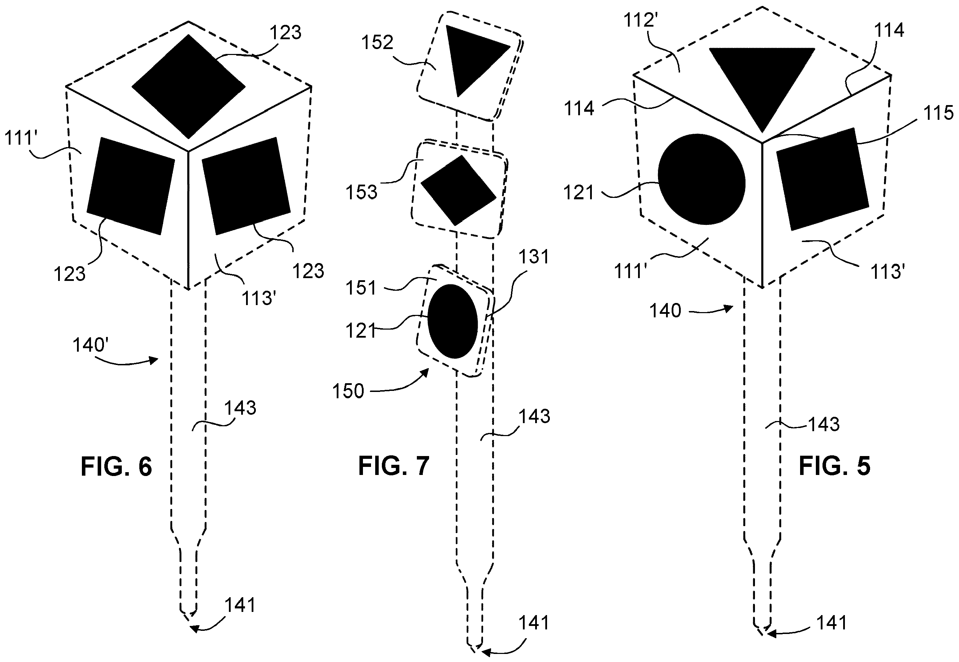

Fig. 5 shows a schematic front side view of the marker device 140 similar to the embodiment as shown in Fig. 1 with different edges 131 compared to the marker body 110 of Fig. 1. Here for example every surface 111', 112' and 113' is quadrilateral shaped but can have any polygonal shape. The outer edge is not necessarily in parallel to the delimiting edge. The three surfaces still meet at the vertex 115. The marker device 140 shown with dashed lines indicates that the smaller tip 141 and the larger rod 143 of the marker device 140 can be of different dimensions, especially longer, shorter, thicker or thinner.

Fig. 6 shows a schematic front side view of a further marker device 140' with three identical markings 123. In short, marker device 140' can be identical to marker device 140 in view of the rhombus-shaped surfaces 111', 112', 113' with the proviso that the three marker symbols are all marker symbols 123, i.e. squares. Of course, this indicates that in other embodiments of the marker device the marker symbols, which can be identical, can also be identical 2D barcodes or other symbols.

Fig. 7 shows a schematic front side view of a marker device 150 with an in-line distribution of the marking surfaces 151, 152 and 153. Here, three marking surfaces are positioned on the rod 143, one above the other with an orientation of each surface being identical to the orientation of the surfaces of the marker device 100 of Fig. 1 or the marker device 140 of Fig. 5. The main point is that the orientation of the surfaces 151, 152 and 153 are similarly different one to each other as for the other marker devices. It is preferable that the square-formed surfaces are not positioned too far away one from the other to avoid a loss of space for the user to grip the rod 143 or to be obliged to provide a far longer rod 143, which is detrimental for the precision of the calculation of the position of the tip 141 in relationship to the determined points of the symbols on the marker surfaces.

Fig. 8A shows a schematic front side view of a marker device 160 with an attachment portion 161. The attachment portion 161 is a hollow cylindrical sleeve with the longitudinal slit allowing for a clamping of positioning sticks, drill guides or other medicinal devices in the central opening of the attachment portion 161. Of course, other attachment portion 161 can be provided adapted to be attached to further different medicinal devices.

Fig. 8B shows the marker device 160 of Fig. 8A rotated by an angle in a forward rotation. Then, the edge 131 of the marker body appears at the outside edges of marker surface 113 shown as double dashed lines. Similar edges appear in the further views Figs. 9 and 10. As explained above, dashed lines are used to show that these body contours do not need to be following straight lines but can be chosen differently.

Fig. 9 shows the marker device 160 of Fig. 8A rotated by an angle in a counterclockwise rotation. Fig. 10 shows the marker device 160 of Fig. 8A rotated by the same angle value in a clockwise rotation. The angle β (beta) in Figs. 9 and 10 indicates the dihedral angle between the top surface and the respective side surface of the trihedron. The marker surfaces 111, 112 and 113 are facing away from each other resembling an umbrella shape.

In this case the angle β (beta) would be between 181 to 315 degrees, preferably 205 to 235 degrees. In the embodiment of Figs. 8A to 10, this angle β (beta) is 225 degrees. The angle α (alpha) in Fig. 8B indicates the polygonal angle at the vertex 115 of the surface looked at. The angle α (alpha) can be chosen between 45 degrees and 179 degrees, preferably 110 degrees to 125 degrees. In the embodiment of Figs. 8A to 10, this angle α (alpha) is 120 degrees.

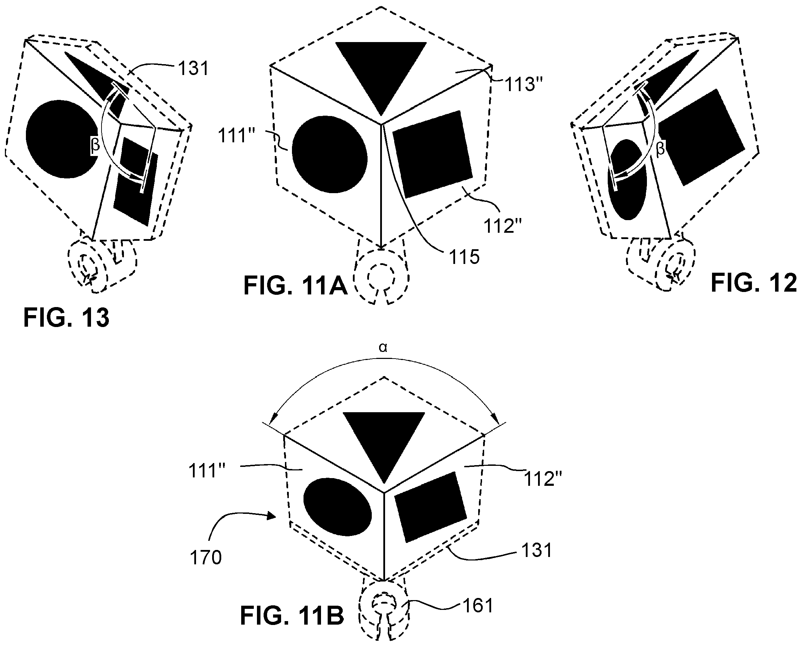

Fig. 11A shows a schematic front side view of a marker 170 with the same attachment portion 161 as before. Fig. 11B shows the marker of Fig. 11A rotated by an angle in backward rotation. Fig. 12 shows the marker of Fig. 11A rotated by an angle in a counterclockwise rotation. Fig. 13 shows the marker of Fig. 11A rotated by the same angle in a clockwise rotation. The angle β (beta) in Figs. 12 and 13 indicates the dihedral angle between the top surface and the respective side surface of the trihedron. The marker surfaces 111", 112" and 113" are facing towards each other resembling an upturned umbrella shape. In this case the angle β (beta) would be between 45 to 179 degrees, preferably 125 to 155 degree. In the embodiment of Fig. 11A to 13, this angle β (beta) is 135 degrees. The angle α (alpha) in Fig. 11B indicates the polygonal angle at the vertex 115 of the surface looked at . The angle α (alpha) can be chosen between 45 degrees and 179 degrees, preferably 110 degrees to 125 degrees. In the embodiment of Fig. 11A to 13, this angle α (alpha) is 120 degrees.

In fact the views as shown in these drawings Fig. 8A to Fig. 13 are more or less POV-shots, i.e. showing the view of the user 50 holding the marker device in one hand (point-of-view shot as technique in motion photography).

The embodiments explained above use a three-surface marker device 160 or 170. It is possible to use a different number of marker surfaces, below marked as 176, 181 and 186. Fig. 14 shows a schematic view from above on a further marker device 175 having two marking surfaces 176; Fig. 15 shows a schematic view from above on a further marker device 180 having four marking surfaces 181; and Fig. 16 shows a schematic view from above on a further marker device 185 having five marking surfaces 186. Here, only Fig. 14 shows an attachment portion 161, but they can be provided or integrated with a pen and rod as in the embodiments described above.

Fig. 14 shows two marker surfaces 176 with a partial rounded edge 178 and "curve and point" marker symbols. Fig. 15 shows four marker surfaces 181 with a rounded edges 183 and different hollow marker symbols 182. Fig. 16 shows five marker surfaces 181 with edges 138/139 as shown for some of the three marker surface marker devices as shown above and 2D barcode marker symbols 187.

It is clear that the different marker symbols can be exchanged in different number-of-marker-surfaces devices and the edges can be chosen accordingly. The four-surface marker device 180 has regular angles of 90 degrees between delimiting edges 114 whereas the five-surface marker device 185 has different value of angles between about 45 degrees to almost 90 degrees, whereas in the view from above, an regular angle of 72 degrees would be possible too.

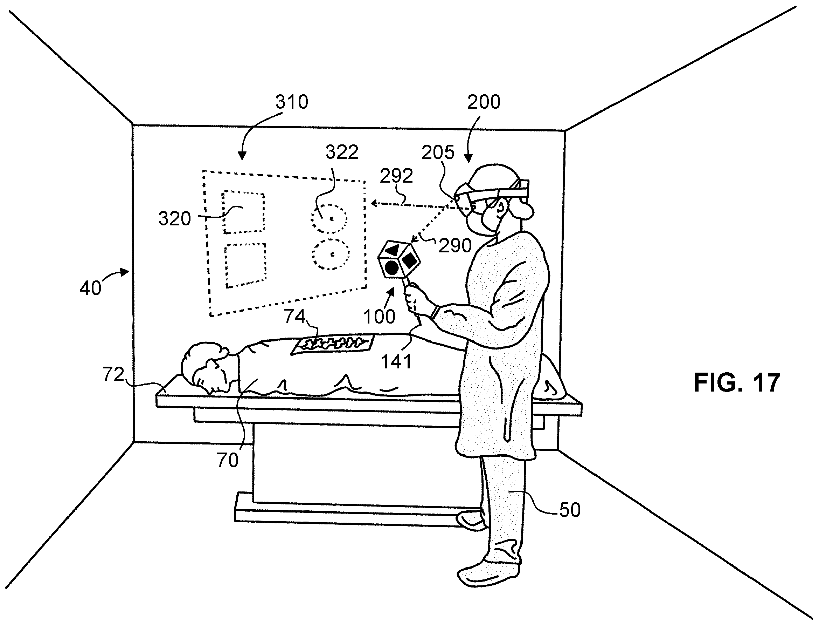

Fig. 17 shows the user 50 of Fig. 1 in an operating room with a patient 70 lying on his stomach with his dorsal vertebrae 74 visible on an operating table 72. The surgeon 50 has an augmented reality head-mounted display 200 on his head with a monocular front camera 205 wherein this camera has a line of sight 290 towards the marker device 100. This LOS would be two converging lines in the case of a binocular camera mounted on such a head-mounted display.

The surgeon has the possibility to look in the direction of his left hand to position the tip 141 of the marker device 100 on a selected vertebrae 74 of the patient 70 to scan and register the shape of the vertebrae. The surgeon 50 can also look up in a clearly different direction 292 compared to the line of sight 290 of the camera 205. In this case, when this different direction 292 is sufficiently different from the line of sight 290 the AR HMD 200 is projecting a virtual image 310 onto the display of the AR HMD 200 which image is shown in Fig. 17 as seen by the surgeon 50 being somewhere in the operating room 40.

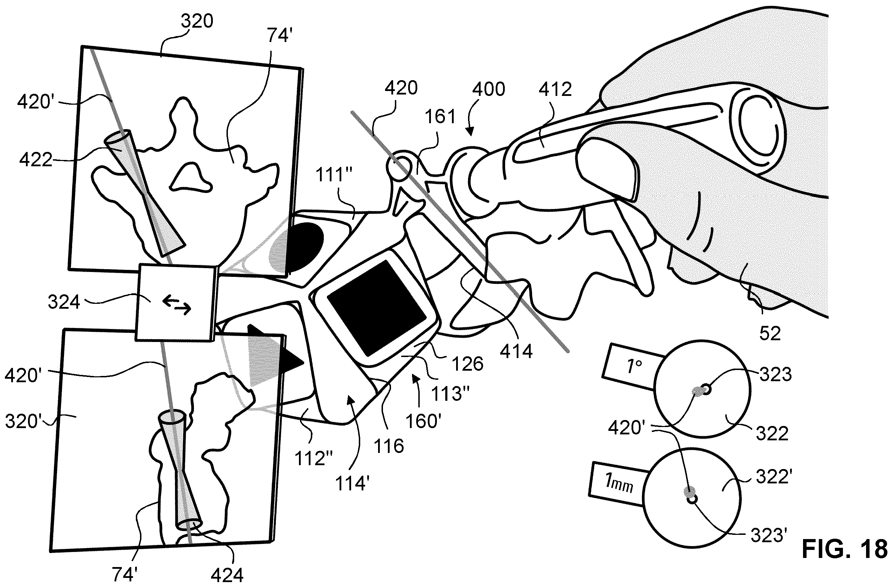

The squares 320 and 320' can show two different virtual reality images of the vertebrae 74 as will be explained in connection with Fig. 18. The round shapes 322 and 322' are virtual reality visualizations providing navigational information or rotational / directional and positional error.

Fig. 18 shows the view of the user 50 through the augmented reality head-mounted display (AR-HMD) 200 holding a drill guide 400 with an attached marker device 160' in his right hand 52 near a dorsal vertebrae 74 and two virtual images of said dorsal vertebrae with the actual axis 420 as determined by the current position and orientation of the drill guide 400. The actual axis 420 is the intended drill axis. The axis 420 shown in Fig. 15 is a virtual axis and is shown on the AR HMD 200. The marker device 160' is attached using the attachment portion 161 with the guide sleeve 414 of the drill guide 400. It is also possible to provide the drill guide 400 with the marker body 110 in one piece.

The marker device 160' is slightly different to the marker device 160 from Fig. 8A in view of the outer form of the marker body 110. The usual delimiting edges 114 have been replaced by a delimiting area 114' having a rounded nature from the vertex 115 to the edge 131 of the marker by the 110. In other words, the joining area between two adjacent marker surfaces are rounded. At the same time each marker surface 111", 112", 113" is built as a recess vis-à-vis the delimiting area 114'. Therefore, the marker surface 111", 112", 113" have a well-defined L- shaped shoulder 116 allowing the fixation of symbol plates 126 on these surfaces in the well determined manner.

The representation of a virtual image 320 in the HMD 200 is a preferred additional optional part of the technical effect of the invention. The embodiment shown in the view of a user 50 in Fig. 18 comprises a first view 320 and the second view 320', showing the above-mentioned intended drill axis 420 in a further virtual presentation 420' from two different directions, usually an axial and a sagittal view. These views are created based on the data gathering with the marker device 160 over the bone surface, i.e. they are created beforehand. Between the two virtual representations 320 and 320' is provided a switch button 324 in the virtual image. This switch button 324 can be actuated by clicking on its virtual representation with a finger in the air, e.g. a gesture, or by voice command to just switch the location side of the virtual representations 320 and 320'. The first view 320 and the second view 320' also comprise the representation of the vertebrae 74'. The representation of the vertebrae has received the reference numeral 74', since it is a virtual representation based on the scanned data with the tip 141 and the registered bone mesh. The bone mesh originates from a 3D imaging modality, e.g. CT. The advantage of the registered vertebrae 74 and its virtual representation 74' is the possibility of the control unit to calculate a safe double conical frustum 422.

Having two views from different directions allows a better visualisation of the real orientation of the drill guide axis, since it can be seen that the virtual drill axis 420' is perfectly aligned in the lower virtual image but is out of the middle in the upper virtual image. At the same time, it is possible to use the lower surface 424 of the conical frustum or another well-defined surface as deepest advance for the drill.

Since the images are virtual representations and calculated conical frustums, it is possible to represent the deviation from a perfect central alignment in the two additional virtual representations 322 and 322'. The center axis 323 of the frustum is shown in a view from above on the frustum. The upper image 322 shows the deviation in degrees whereas the lower representation 322' shows a distance. The deviation in degrees is visualized in that the top of the intended drill axis 420 is shown in the virtual representation 420' to be 1 degree out of the center axis 323 of the frustum and is therefore not in the center of the image. The distance between the virtual representation 420' and the center with the reference numeral 323' is the distance of the currently recorded bore line to the entry point 323' of the registered center axis 323 of the cone on the vortex in millimeters, here equally shown as being 1 mm. The predetermined entry point 323' is the point on the surface of the vertebra 74 where the center axis "comes out". In other words, exactly where the surgeon 50 would optimally place the drill. The center axis 323 is predetermined by the surgeon when planning the intervention based on the further modality images, usually previously acquired CT images of the bone to be operated. The data relating to this virtual planned entry point 323' as well as the direction of drilling, characterized by the virtual center axis of the frustum 323, are predetermined by the surgeon and uploaded to the control unit 600 in advance of the intervention. The data can comprise, beside the frustum 422 also an end point area 424, which is shown in Fig. 18 as bottom surface of the frustum. Such an end point area 424 is the planned depth of the drilling and can be visualized through positional information about the advance of the drill when attached to the holder of the drill guide 400.

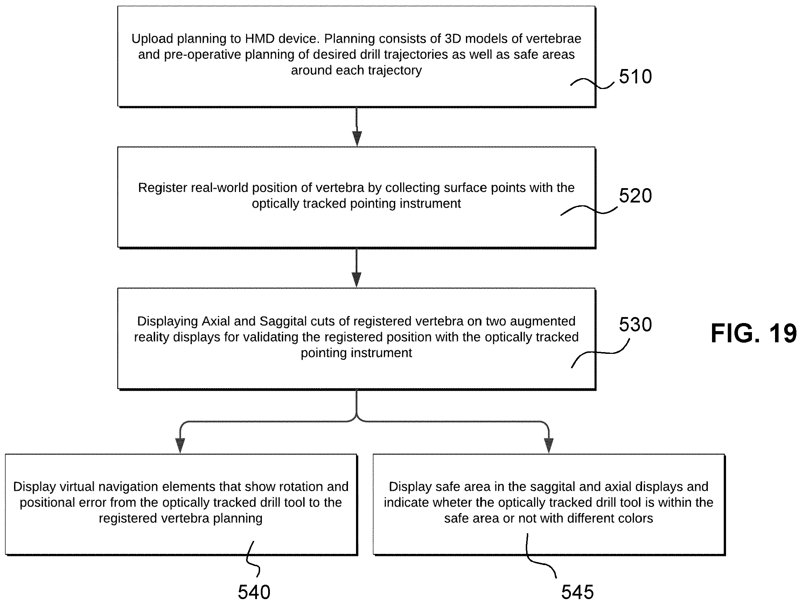

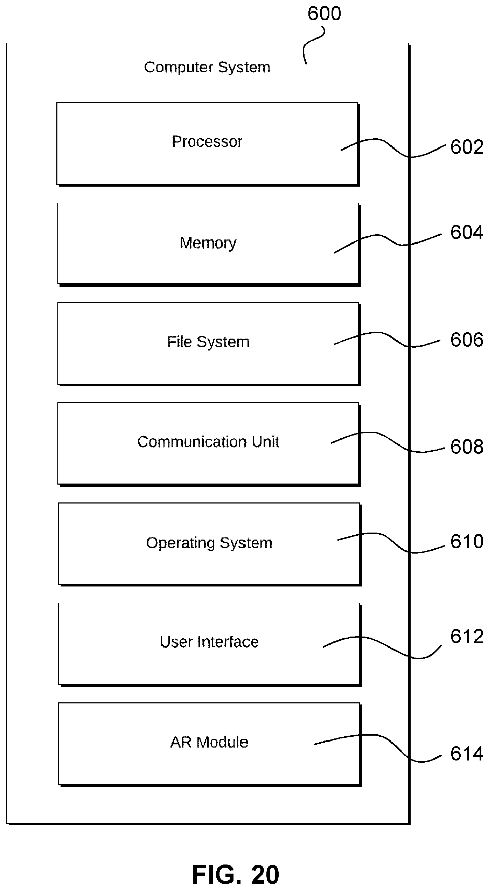

Fig. 19 shows a flowchart of a method for using the system according to the invention; and Fig. 20 shows an overview of a computer system used in connection with such a marker system, especially to execute a method as shown in Fig. 19.

A method for using the system according to the invention comprises a planning step 510. This planning step comprises an upload of the planning to the head mounted display 200. The planning itself consists of 3D models of vertebrae and preoperative planning of desired drill trajectories as well as the save areas around each trajectory. In other words, the upload comprises the creation of the double conical frustum based on the physical structure of the vertebrae. At the same time, it is intended that the guide direction is pleaded to mind based on the physical constraints of the vertebrae of the patient and the need of fixation.

The registration step 520 follows in which the real world position of vertebrae are registered by collecting surface points with the optically correct pointing instrument, i.e. the marker device 160. After having effected that step 520, enough information is collected to create the display as in Fig. 18, i.e. providing the virtual representations based on the planning data at the place of the real vertebrae on the display.

The display step 530 comprises displaying axial and sagittal cuts of registered vertebrae 74 on two augmented reality display areas 320 and 320' for validating the registered position with the optically tracked pointing instrument, i.e. marker device with tip 141. The virtual placed vertebra (registration) is shown in the axial and sagittal slice. User 50 can then put the pointer tip 101 on the real vertebra to see if the axial and sagittal slices with the shown tracked pointer tip is at the same position on the virtual registered vertebra as it is placed on the real world vertebra. This step (done by the user 50) is called validation.

The display step 540 shows virtual navigation elements to represent rotation and positional error from the optically tracked drill tool to the registered vertebrae planning. A further result of the display step 545 showing safe areas in the sagittal and axial displays and indicate whether the optically tracked route is within the safe area or not with different colours.

The control unit 600 as shown in Fig. 20 comprises a computer system having a processor 602, a memory 604, a file system 606, a communication unit 608. It is controlled by an operating system 610 and has one or more user interfaces 612 and an AR module 614. The control unit 600 can be realised at least partly in the AR HMD 200 and partly in an external computer which can be accessed by conventional means as keyboard, mouse, touch screen and allows uploading any pre-obtained patient data. Patient data comprises segmented bone meshes, drill trajectories for each screw that should be navigated as well as the cone/frustum data for each drill trajectory. The control unit comprises a software in its file repository configured to calculate the actual surface touched by the tip 141 based on the captured symbols. The user interfaces 612 may comprise gesture control and voice commands. The AR module 614 controls the representation of the virtual image in the AR HMD 200.

### LIST OF REFERENCE SIGNS

| | | | |
|---|---|---|---|
| 40 | operating room | 135 | outside socket contact |
| 50 | surgeon | 136 | opening |
| 52 | right hand | 138 | base line |
| 60 | bone | 139 | side edge |
| 70 | patient | 140 | marker device |
| 72 | operating table | 140' | marker device |
| 74 | dorsal vertebra | 141 | tip of marker device |
| 74' | virtual representation of dorsal vertebra | 143 | rod of marker device |
| | | 150 | marker device |
| 80 | positioning stick | 151 | first marker surface |
| 100 | marker device | 152 | second marker surface |
| 101 | tip of marker device | 153 | third marker surface |
| 102 | holder of marker device | 160 | marker device (umbrella like marker surfaces) |
| 110 | marker body | | |
| 111 | first marker surface | 160' | marker device |
| 111' | first marker surface | 161 | attachment portion |
| 111" | first marker surface | 170 | marker device (upturned umbrella like marker surfaces) |
| 112 | second marker surface | | |
| 112' | second marker surface | | |
| 112" | second marker surface | 175 | two surface marker device |
| 113 | third marker surface | 176 | marker surface(s) |
| 113' | third marker surface | 177 | marker symbol (curve and point) |
| 113" | third marker surface | | |
| 114 | delimiting edge | 178 | partial rounded edge |
| 114' | delimiting area | 180 | four surface marker device |
| 115 | vertex | 181 | marker surface(s) |
| 116 | shoulder | 182 | hollow shape marker symbol |
| 121 | first marker symbol | 183 | rounded edge |
| 122 | second marker symbol | 185 | five surface marker device |
| 123 | third marker symbol | 186 | marker surface(s) |
| 130 | marker symbol carrier | 187 | 2D barcode marker symbol |
| 131 | edge of marker body | 200 | AR HMD |
| 132 | underside | 201 | strap |
| 133 | middle socket contact | 202 | AR display of HMD |
| 134 | corner socket contact | 205 | camera of HMD |
| 290 | line of sight | 420' | virtual representation of the intended drill axis |
| 292 | direction of view | | |
| 600 | control unit | 422 | double conical frustum |
| 310 | virtual user interface | 424 | end point area |
| 320 | virtual image, view 1 | 510 | planning upload step |
| 320' | virtual image, view 2 | 520 | registering step |
| 322 | virtual navigation indicator (angle) | 530 | display step |
| | | 540 | display step |
| 322' | virtual navigation indicator (distance) | 545 | display step |
| | | 600 | control unit |
| 323 | frustum center axis | 602 | processor |
| 323' | planned entry point | 604 | memory |
| 324 | switch button | 606 | file system |
| 400 | drill guide | 608 | communication unit |
| 412 | holder | 610 | operating system |
| 414 | guide sleeve | 612 | user interfaces |
| 420 | intended drill axis | 614 | AR module |

## Claims

1. Marker system comprising:
- a marker device (100, 140, 150, 160, 170, 175, 180, 185) having a marker body (110) with two to five marker surfaces (111, 112, 113; 111', 112', 113'; 111", 112", 113"; 151, 152, 153; 176; 181; 186) wherein each two of these marker surfaces have a common edge (114) wherein the planes of these marker surfaces meet at said common edge (114) at a blunt angle, wherein each marker surface comprises a marker symbol (121, 122, 123; 177; 182; 187),
- an augmented reality head-mounted display (200) for a person (50) incorporating a camera (205),
- a control unit (600) connected to the augmented reality head-mounted display (200) and the camera (205), wherein the camera (205) is mainly aligned in the user's line of sight (290) and wherein the control unit is configured to calculate the position and orientation (420) of the marker device based on receiving images from the camera (205) including the marker symbols (121, 122, 123).

2. Marker system according to claim 1, wherein the marker device has a tip (101, 141) at the end of a rod (143) defining the position of the tip (101, 141) vis-à-vis the marker surfaces (111, 112, 113).

3. Marker system according to claim 2, wherein the marker device has three marker surfaces (151, 152, 153) positioned one above the other in longitudinal direction on the rod (143).

4. Marker system according to claim 1, wherein the marker device has a holder (133, 134, 135; 136) defining a predetermined direction vis-à-vis the marker surfaces (111, 112, 113).

5. Marker system according to claim 4, wherein the holder comprises one or more positioning sockets (133, 134, 135; 136) at the underside (132) of the marker body (110), wherein the positioning socket(s) is/are configured to accommodate a positioning stick (80) defining a predetermined positioning direction vis-à-vis the three marker surfaces (111, 112, 113).

6. Marker system according to any one of claims 1 to 5, wherein the blunt angle at which the planes of these marker surfaces meet at said common edge (114) is greater than 180 degrees and the planes create an umbrella like shape, or is smaller than 180 degrees and the planes create an upturned umbrella like shape.

7. Marker system according to any one of claims 1 to 6, wherein the dihedral angle between the top surface and the respective side surface of the polyhedron is between 205 degrees and 235 degrees, preferably 225 degrees for the umbrella like shape or between 125 degrees and 155 degrees, preferably 135 degrees for the upturned umbrella like shape, wherein the polygonal angle at the vertex (115) is between 110 degrees and 125 degrees, preferably 120 degrees.

8. Marker system according to any one of claims 1 to 7, wherein the control unit is configured to access a previously acquired 3D image of a body (74'), to calculate and to display said 3D image (74') in at least one of an axial and a sagittal view in an side area (320, 320') of the field of view of the augmented reality head-mounted display (200), to calculate and display a virtual representation of the position and orientation of the marker device (420').

9. Marker system according to claim 8, wherein the control unit is configured to display a previously determined shape of intended intervention (422) in the said view(s) of the side area (320, 320') together with the virtual representation of the position and orientation of the marker device (420').

10. Marker system according to claim 9, wherein the previously determined shape of intended intervention is a double conical frustum (422).

11. Marker system according to claim 10, wherein the control unit is configured to calculate and to display the virtual representation of the marker device (420') in a view from above on the conical frustum (422), providing navigational information or rotational / directional and positional error in a further side area.

12. Marker system according to claim 11, wherein the view(s) from above comprise(s) a center indication representing the frustum axis (323) and/or the virtual planned entry point (323') and the virtual representation of the marker device (420') reflecting the angle or distance deviation from the frustum axis (323) and the virtual planned entry point (323'), respectively.

13. Marker system according to any one of claims 1 to 12, wherein the marker symbols are from the group comprising 2D barcodes, filled or line polygons, identical or different geometrical shapes.

14. Method of providing visual indication for a planned drill entry point (323') into a body (74) and planned drill direction (323) comprising:
- having a marker system according to any one of claims 1 to 13,
- having a 3D image modality (74') of the body (74),
- determining the planned drill direction (323) and planned drill entry point (323') in the body (74) as virtual data,
- registering real world position of the body (74) by collecting surface points with a marker device of the marker system,
- displaying axial and sagittal cuts of the 3D image modality (74') of the body (74) for validating the registered position with the marker device of the marker system on an augmented reality head-mounted display (200) of the marker system, and
- displaying virtual navigation elements reflecting rotation (420' to 322) and distance (420' to 322') error from the predetermined planned drill direction (323) and planned drill entry point (323') on the augmented reality head-mounted display (200) of the marker system.

15. Method according to claim 14, wherein the determination of the planned drill direction (323) and planned drill entry point (323') in the body (74) as virtual data comprises the step of providing a virtual double conical frustum (422) to be displayed in connection with the axial and sagittal cuts of the 3D image modality (74').
